# EUROPÄISCHE PATENTANMELDUNG

(11) **EP 4 707 806 A1**
(43) Veröffentlichungstag der Anmeldung: **11.03.2026**
(21) Anmeldenummer: 24198554.8
(22) Anmeldetag: 05.09.2024
(51) Int. Cl.: G01N 33/543, B01L 3/00, B82Y 5/00

(54) **VERFAHREN UND SYSTEM ZUR DETEKTION VON ZELLEN IN EINER BIOPSIEPROBE**

(71) Anmelder: Friedrich-Alexander-Universität Erlangen-Nürnberg, in Vertretung des Freistaates Bayern, 91054 Erlangen (DE)
(72) Erfinder: Nagy, Roland, 90765 Fürth (DE); Schneider-Stock, Regine, 91301 Forchheim (DE); Tietze, Rainer, 91074 Herzogenaurach (DE)
(74) Vertreter: Lösch, Christoph Ludwig Klaus

(57) **Zusammenfassung**

Die Erfindung betrifft ein Verfahren und System zur Detektion von Zellen einer ersten Zellart (1) in einer Biopsieprobe, in der eine Mehrzahl von Zellen unterschiedlicher Zellarten (2, 3) vorhanden sind. Ferner betrifft die Erfindung eine Vorrichtung zur Durchführung einer Magnetfeldmessung an mit magnetischen Nanopartikeln markierten Zellen einer Biopsieprobe. Das Verfahren weist folgende Verfahrensschritte auf: Durchführen einer Gewebedissoziation der Biopsieprobe zum Vereinzeln der in der Biopsieprobe enthaltenen Zellen; Zuführen von magnetischen Nanopartikeln (11) zu den vereinzelten Zellen zum selektiven Markieren der Zellen der ersten Zellart (1); Entfernen der überflüssigen magnetischen Nanopartikel (11); Einführen der Zellen in einen Mikrokanal (5), wobei der Mikrokanal (5) einen Querschnitt aufweist, der den gleichzeitigen Durchgang lediglich einer markierten Zelle (1) erlaubt; Durchführen einer Magnetfeldmessung im Mikrokanal (5) mittels eines auf dem Farbzentrum-Prinzip basierenden Quantenmagnetfeldsensors (6); und Aussortieren der markierten Zellen (1) nach dem Durchlaufen des Mikrokanals (5).

## Beschreibung

Die Erfindung betrifft ein Verfahren und System zur Detektion von Zellen einer ersten Zellart in einer Biopsieprobe, in der eine Mehrzahl von Zellen unterschiedlicher Zellarten vorhanden sind. Ferner betrifft die Erfindung eine Vorrichtung zur Durchführung einer Magnetfeldmessung an mit magnetischen Nanopartikeln markierten Zellen einer Biopsieprobe.

Eine oft anzutreffende Aufgabe in der Medizintechnik liegt darin, veränderte, auffällige oder krankhafte Zellen in einer aus einem Lebewesen entnommenen Gewebeprobe zu identifizieren. Ein allgemein bekanntes Verfahren zur Untersuchung von Biopsieproben stellt die histologische Schnittuntersuchung dar. Allerdings sind beispielsweise im Kontext von Tumorerkrankungen sensitive, schnelle und zuverlässige Untersuchungen nötig, um eine möglichst eindeutige und schnelle Diagnose zu liefern. Eine Herausforderung besteht hierbei darin, dass in einer Biopsieprobe (z.B. im Rahmen einer Erstdiagnose auf verschiedene Tumorerkrankungen) sehr wenige Tumorzellen enthalten sein können. Diese sollten jedoch mit einer hohen Zuverlässigkeit identifiziert werden. Zugleich soll das Ergebnis der Untersuchung im Allgemeinen möglichst schnell vorliegen, um beispielsweise den ärztlichen Eingriff unmittelbar in Abstimmung mit dem Untersuchungsergebnis durchzuführen. So wird die Entnahme von Gewebeproben oft während einer Operation angefordert, wenn der Chirurg kurzfristige Informationen benötigt, um den weiteren Verlauf und Umfang des Eingriffs zu bestimmen. Ein häufiges Beispiel ist die Überprüfung, ob Lymphknoten von einem Tumor betroffen sind oder ob ein Tumor vollständig entfernt wurde.

Der Erfindung liegt die Aufgabe zugrunde, ein sensitives, zuverlässiges und zugleich schnelles Verfahren und System zur Detektion von Zellen einer ersten Zellart in einer Biopsieprobe, in der eine Mehrzahl von Zellen unterschiedlicher Zellarten vorhanden sind, anzubieten. Ferner ist es Aufgabe der Erfindung, eine Vorrichtung anzubieten, welche eine sensitive, zuverlässige und zugleich schnelle Durchführung einer Magnetfeldmessung an mit magnetischen Nanopartikeln markierten Zellen einer Biopsieprobe erlaubt, um Zellen einer ersten Zellart in einer Biopsieprobe zu detektieren.

Diese Aufgabe wird durch ein Verfahren mit den Merkmalen des Anspruchs 1, durch eine Vorrichtung mit den Merkmalen des Anspruchs 11 sowie durch ein System mit den Merkmalen des Anspruchs 15 gelöst. Vorteilhafte Ausführungsformen sind in den Unteransprüchen beschrieben.

Das erfindungsgemäße Verfahren zur Detektion von Zellen einer ersten Zellart in einer Biopsieprobe, in der eine Mehrzahl von Zellen unterschiedlicher Zellarten vorhanden sind, weist folgende Verfahrensschritte auf: Durchführen einer Gewebedissoziation der Biopsieprobe zum Vereinzeln der in der Biopsieprobe enthaltenen Zellen; Zuführen von magnetischen Nanopartikeln zu den vereinzelten Zellen zum selektiven Markieren der Zellen der ersten Zellart; Entfernen der überflüssigen magnetischen Nanopartikel; Einführen der Zellen in einen Mikrokanal, wobei der Mikrokanal einen Querschnitt aufweist, der den gleichzeitigen Durchgang lediglich einer markierten Zelle erlaubt; Durchführen einer Magnetfeldmessung im Mikrokanal mittels eines auf dem Farbzentrum-Prinzip basierenden Quantenmagnetfeldsensors; und Aussortieren der markierten Zellen nach dem Durchlaufen des Mikrokanals.

Unter Zellen einer Zellart werden hierbei Zellen verstanden, die sich durch eine Eigenschaft von den Zellen der anderen Zellarten unterscheiden. Beispielsweise können Tumorzellen einer ersten Tumorzelllinie eine erste Zellart bilden, Tumorzellen einer zweiten Tumorzelllinie eine zweite Zellart bilden und nichtkanzerogene Gewebezelle eine dritte Zellart bilden.

Ferner kann auch eine Detektion von bestimmten Viren (anstatt von Zellen) erfolgen. Dabei können beispielsweise Viren (z.B. Corona-Viren) eines ersten Typus eine erste Virenart bilden, Viren eines zweiten Typus eine zweite Virenart, die wie bei Zellen beschrieben magnetisch markiert werden und dann erfindungsgemäß analog detektiert werden.

Der in den Ansprüchen beschriebene Gegenstand der Erfindung bezieht sich auf Zellen aller Art und kann sich alternativ auch auf Viren aller Art beziehen.

Die Detektion unterschiedlicher Zelltypen ist ferner nicht auf das Gebiet der Onkologie beschränkt. So könnten ferner als weitere Anwendung Biomarker erfasst werden, die auf neurodegenerative Erkrankungen hindeuten. Hierzu können SPIONs mit Antikörpern, die in Korrelation zu solchen Erkrankungen wie Anti-TAU-Antikörper oder Anti Amyloid-Beta Antikörper oder Anti Lewybody Antikörper stehen, funktionalisiert werden. Über die Kopplung definierter Oberflächenmarker der Zellen der ersten Zellart mit magnetischen Nanopartikeln kann die magnetische Signatur dieser Zellen (z.B. Tumorzellen) über Quantenmagnetometrie hoch effizient und spezifisch ausgelesen werden. Mit Hilfe der Quantensensorik können Parameter wie die magnetische Feldstärke in verschiedenen physikalischen, biologischen oder materiellen Systemen bestimmt werden. Sie zielt insbesondere darauf ab, die Präzision und Selektivität bei Messungen durch Quanteneffekte zu optimieren. Quantenmagnetfeldsensoren, die auf dem Farbzentrum-Prinzip (z.B. NV-Zentrum im Diamanten) basieren, haben folgende vorteilhafte Eigenschaften: hohe magnetische Empfindlichkeit, Betrieb bei Raumtemperatur, großer Dynamikbereich (mehrere Tesla) und kalibrierungsfreien Betrieb.

Die Durchführung der Magnetfeldmessung im Mikrokanal ermöglicht die zellgenaue Detektion, was wiederum zu einer hohen Selektivität und Sensitivität der Messung führt.

Mit anderen Worten wird ein Quantenmagnetfeldsensor genutzt, um selektiv Zellen einer bestimmten Zellart (z.B. Zellen einzelner Krebszelllinien) zu bestimmen. D.h. über die selektive Bindung der magnetischen Nanopartikel an Zellen und des daraus resultierenden Magnetfelds wird erkannt, welche Zellen markiert sind und welche nicht. Da nur Zellen markiert wurden, die zu einer bestimmten Zellart gehören, kann über das Magnetfeld und der Aussortierung nach dem Durchlauf des Mikrokanals diese Zellen selektiv bestimmt werden.

Damit ermöglicht das erfindungsgemäße Verfahren eine sensitive, zuverlässige und schnelle Detektion von Zellen einer ersten Zellart in einer Biopsieprobe. Beispielsweise ermöglicht das erfindungsgemäße Verfahren die Unterbrechung einer laufenden Operation zur histologischen Untersuchung für nur wenige Minuten, während bislang die Operation über einen längeren Zeitraum, oft für mehrere Stunden, unterbrochen werden musste. Hierbei kann die Biopsieprobe eine unter Narkose entfernte Gewebeprobe (Tumorrandbestimmung) sein.

Die Biopsieprobe kann ferner in vorteilhafter Weise eine Feinnadelbiopsieprobe sein. Bei der Feinnadelaspiration wird eine feine Nadel in abnormes Gewebe oder verdächtige Körperflüssigkeiten eingeführt. Die Feinnadelbiopsie gilt als wenig invasiv, liefert jedoch auch weniger Probenmaterial als herkömmlich Biopsieproben. Aufgrund der hohen Sensitivität des erfindungsgemäßen Verfahrens wird nur eine geringe Untersuchungsmenge benötigt. Entsprechend eignet sich eine Feinnadelbiopsieprobe hier sehr gut.

Die magnetischen Nanopartikel sind vorteilhafter Weise als superparamagnetische Eisenoxid-Nanopartikel (engl.: super paramagnetic iron oxide nanoparticles - SPIONs) ausgebildet. Diese besitzen Vorteile durch kleine Partikelgrößen, eine magnetische Steuerbarkeit, Biokompatibilität, die Möglichkeit vielfältiger Oberflächenmodifikationen und kolloidale Stabilität.

In einer vorteilhaften Ausführungsform kann das Verfahren ferner einen Verfahrensschritt zum Verändern des Querschnitts des Mikrokanals in Abhängigkeit von der Größe der Zellen aufweisen. Auf diese Weise kann das Einsatzgebiet vergrößert werden, da für verschiedene Zellgrößen sichergestellt werden kann, dass lediglich eine Zelle gleichzeitig in den Mikrokanal eintreten kann.

Am Eingang des Mikrokanals kann ein Durchflussregler angeordnet sein. Auf diese Weise ist sichergestellt, dass nur eine begrenzte Anzahl an Zellen in den Mikrokanal eintritt. Die Magnetfeldmessung kann dadurch störungsfreier ablaufen.

Am Ausgang des Mikrokanals kann ein Mikrofluidschalter angeordnet werden. Dieser ermöglicht das unmittelbare Aussortieren der markierten Zellen.

In anderen Worten werden die magnetisch identifizierten Zellen am Ausgang des Mikrokanals von den restlichen Zellen durch eine Sortierung getrennt. Hierzu ist für die Implementierung der Mikrofluidik am Eingang des Mikrokanals ein Durchflussregler und am Ausgang ein Mikrofluidschalter zur Zellsortierung angeschlossen werden. Basierend auf der Position der Zelle während der Magnetfeldmessung und dem kalibrierten Zellfluss nach der Messung kann die Position des Mikrofluidschalters zur Sortierung der Zelle festgelegt werden.

Der Querschnitt des Mikrokanals ist so dimensioniert, dass dieser den gleichzeitigen Durchgang lediglich einer markierten Zelle erlaubt, wobei der Querschnitt in vorteilhaften Ausführungsformen rund/oval bzw. quadratisch oder rechteckig ist. Es sind jedoch auch Ausführungsformen mit halbrundem Querschnitt oder ähnlich möglich.

In einer vorteilhaften Ausführungsform weist der Quantenmagnetfeldsensor eine Sensorschicht auf, in der Farbzentren ausgebildet sind und die zumindest einen Teilbereich einer inneren Oberfläche des Mikrokanals bildet. Auf diese Weise kann die Sensorschicht in unmittelbarer räumlicher Nähe zu den zu detektierenden Zellen angeordnet sein. Entsprechend wenig Bauraum wird durch den Quantenmagnetfeldsensor benötigt.

Für die Magnetfeldmessung durch den Quantenmagnetfeldsensor kann eine sog. kontinuierliche Messung (engl. Continous Wave) durchgeführt werden. Diese Art der Messung besitzt einen relativ einfachen Aufbau und liefert besonders schnelle Messergebnisse.

Es ist auch möglich, die die Magnetfeldmessung durch den Quantenmagnetfeldsensor mittels einer sog. gepulsten Messung durchzuführen. Diese Art der Messung ist gegenüber der oben genannten kontinuierlichen Messung um den Faktor 10 bis 100 sensitiver. Sie besitzt jedoch einen komplexeren Aufbau.

Nach dem Aussortieren der markierten Zellen kann als weiterer Verfahrensschritt eine lichtmikroskopische Untersuchung der aussortierten, markierten Zellen durchgeführt werden, um beispielsweise weitere Eigenschaften der aussortierten Zellen zu untersuchen.

Ferner können nach dem Aussortieren der markierten Zellen molekulare Folgeuntersuchungen aller Art durchgeführt werden, z.B. eine Bestimmung des Mutationsstatus der Zellen und ihrer Gensignaturen.

Bei der Untersuchung von Tumorzellen aus Blut könnten auch zirkulierende Tumorzellen aussortiert und damit isoliert werden.

Weitere vorteilhafte Ausführungsformen können darauf abzielen nicht nur eine Zellart auszusortieren, sondern mindestens eine weitere Zellart. So können im Verfahrensschritt "Zuführen von magnetischen Nanopartikeln" erste magnetische Nanopartikel zum selektiven Markieren der Zellen der ersten Zellart und zweite magnetische Nanopartikel zum selektiven Markieren von Zellen einer zweiten Zellart zugeführt werden. Die ersten magnetischen Nanopartikel unterscheiden sich von den zweiten Nanopartikeln hierbei sowohl in ihrer Oberflächenbeschaffenheit (um eine selektive Markierung der Zellen der ersten Zellart bzw. eine selektive Markierung der Zellen der zweiten Zellart zu ermöglichen) als auch in ihren magnetischen Eigenschaften. Während der Magnetfeldmessung im Mikrokanal kann dann zwischen den markierten Zellen der ersten Zellart und den markierten Zellen der zweiten Zellart unterschieden werden. Im Ergebnis kann ein gleichzeitiges, selektives Aussortieren der ersten und der zweiten Zellart erfolgen.

Auch können an den nach dem Verfahrensschritt "Aussortieren der markierten Zellen" verbleibenden Zellen wieder die Verfahrensschritte "Zuführen von magnetischen Nanopartikeln" bis "Aussortieren der markierten Zellen" durchgeführt werden, wobei im Verfahrensschritt "Zuführen von magnetischen Nanopartikeln" dann weitere magnetische Nanopartikel zum selektiven Markieren von Zellen einer weiteren Zellart zugeführt werden. Im Ergebnis kann ein serielles, selektives Aussortieren mehrerer Zellart erfolgen.

Die erfindungsgemäße Vorrichtung zur Durchführung einer Magnetfeldmessung an mit magnetischen Nanopartikeln markierten Zellen einer Biopsieprobe besitzt in ihrer allgemeinsten Form einen Mikrokanal, der einen Querschnitt aufweist, der den gleichzeitigen Durchgang lediglich einer markierten Zelle erlaubt, und einen auf dem Farbzentrum-Prinzip basierenden Quantenmagnetfeldsensor zur Durchführung einer Magnetfeldmessung im Mikrokanal. Diese Vorrichtung eignet sich insbesondere durch Durchführung der Verfahrensschritte "Einführen der Zellen in einen Mikrokanal" und "Durchführen einer Magnetfeldmessung im Mikrokanal".

Das erfindungsgemäße System zur Detektion von Zellen einer ersten Zellart in einer Biopsieprobe, in der eine Mehrzahl von Zellen unterschiedlicher Zellarten vorhanden sind, weist auf: eine Gewebedissoziationseinrichtung zum Durchführen einer Gewebedissoziation der Biopsieprobe zum Vereinzeln der in der Biopsieprobe enthaltenen Zellen; eine Markiereinrichtung zum Zuführen von magnetischen Nanopartikeln zu den vereinzelten Zellen und zum selektiven Markieren der Zellen der ersten Zellart; eine Entfernungseinrichtung zum Entfernen der überflüssigen magnetischen Nanopartikel; die oben genannte Vorrichtung; und eine Aussortiervorrichtung zum Aussortieren der markierten Zellen nach dem Durchlaufen des Mikrokanals.

Die Erfindung ist anhand von Ausführungsbeispielen in den Zeichnungsfiguren weiter erläutert, wobei gleiche Bezugszeichen gleiche bzw. gleichwirkende Komponenten bezeichnen. Es zeigen:
- Fig. 1: ein Blockdiagramm ersten erfindungsgemäßen Verfahrensablaufs;
- Fig. 2: ein Blockdiagramm eines zweiten erfindungsgemäßen Verfahrensablaufs;
- Fig. 3: ein Blockdiagramm eines dritten erfindungsgemäßen Verfahrensablaufs;
- Fig. 4: eine schematische Schnittansicht einer Vorrichtung zur Durchführung einer Magnetfeldmessung und einer Aussortiervorrichtung;
- Fig. 5a bis 5i: Schematische Darstellungen einer Sortierung von Zellen einer Biopsieprobe; und
- Fig. 6a bis 6f: Schematische Schnittansichten eines Mikrokanals mit Quantenmagnetfeldsensor.

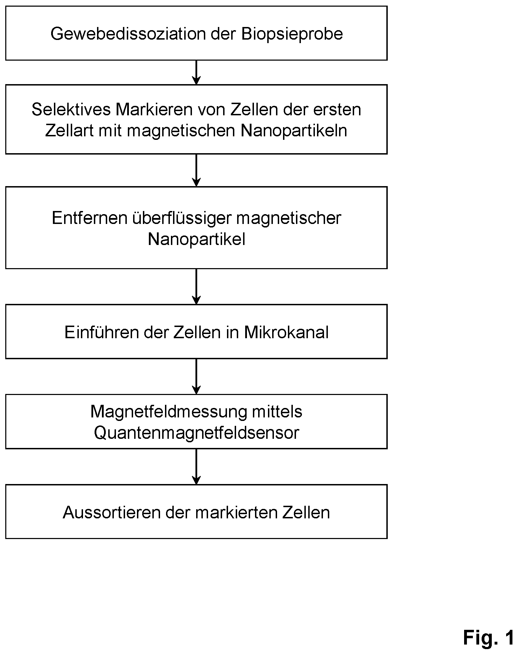

Fig. 1 stellt in Form eines Block- oder Ablaufdiagramms einen ersten erfindungsgemäßen Verfahrensablauf dar. Ausgangspunkt ist eine Biopsieprobe, die beispielsweise im Rahmen einer medizinischen Untersuchung entnommen wurde. Hierbei handelt es sich insbesondere um eine Feinnadelbiopsieprobe. Es kann sich jedoch auch um eine Stanzbiopsieprobe handeln.

Die Biopsieprobe wird einer Gewebedissoziation zum Vereinzeln der in der Biopsieprobe enthaltenen Zellen unterzogen. Verfahren zur Gewebedissoziation sind allgemein bekannt. So werden oft manuelle Methoden angewandt. Vorteilhafter sind jedoch automatisierte Verfahren, die eine effektivere und reproduzierbarere Aufbereitung der Gewebeproben ermöglichen. So sind etwa auch automatisierte Verfahren bekannt, die mechanische Verfahren mit Verfahren der enzymatischen Verdauung kombinieren.

Nachdem die Zellen der Biopsieprobe in vereinzelter Form vorliegen, erfolgt die Zufuhr von magnetischen Nanopartikeln zu den vereinzelten Zellen. Hierbei handelt es sich bei den magnetischen Nanopartikeln um SPIONs, die auf die zu detektierende Zellart abgestimmt sind, sodass ein selektives Markieren der Zellen dieser Zellart (im Folgenden auch erste Zellart oder Targetzellen genannt) erfolgt.

Voraussetzung für die selektive Markierung der Targetzellen mittels SPIONs ist eine entsprechende Oberflächenbeschaffenheit der magnetischen Nanopartikel. Zunächst muss diese so gestaltet werden, dass die Nanopartikel in der entsprechenden Matrix kolloidal stabil sind. Ansonsten kommt es aufgrund der Oswald Reifung zum Anwachsen der Partikel und schließlich zur Sedimentation. Unbeschichtete Nanopartikel sind außerdem zugänglich für unspezifische Adsorptionsvorgänge mit dem Ergebnis, dass sämtliche Zellen magnetisch markiert werden. SPIONs für diesen Anwendungszweck müssen also in erster Linie kolloidal stabil und möglichst bindungsneutral sein. Für die spezifische Bindung an die Targetzellen muss zunächst die Expression entsprechender Zellrezeptoren bekannt sein. Diese sollen entweder exklusiv nur auf den Zellen der ersten Zellart vorhanden sein oder im Vergleich zu den Zellen der weiteren Zellarten deutlich überexprimiert sein. Dann werden entsprechende Liganden (also Antikörper gegen diese Rezeptoren) ausgewählt und an die Oberfläche der SPIONs gebunden. Dies kann entweder unspezifisch gelingen z.B. über eine Aktivesterbindungsstrategie, um die immer vorhandenen Aminogruppen im Antikörperprotein zu adressieren oder über chemoselektives Tagging, wenn z. B. spezielle funktionelle Targets vorab in diesem Protein implementiert wurden. Beispiel für letzteres ist das komplementäre Biotin-Streptavidin Bindungspaar. Es gibt aber vielzählige andere Orthogonale (also gerichtete) Bindungsstrategien auf Basis unterschiedlicher Tags. Beispiele sind Snap-Tag, Spy-Tag usw.

Überflüssige Nanopartikel - also Nanopartikel, die sich nicht mit den Zellen der ersten Zellart verbunden haben - werden in einem anschließenden Reinigungs- bzw. Auswaschschritt entfernt. Übrig bleiben damit vereinzelte Zellen, wobei die Zellen der ersten Zellart mit magnetischen Nanopartikeln markiert sind, während die Zellen der restlichen Zellarten nicht mit magnetischen Nanopartikeln markiert sind.

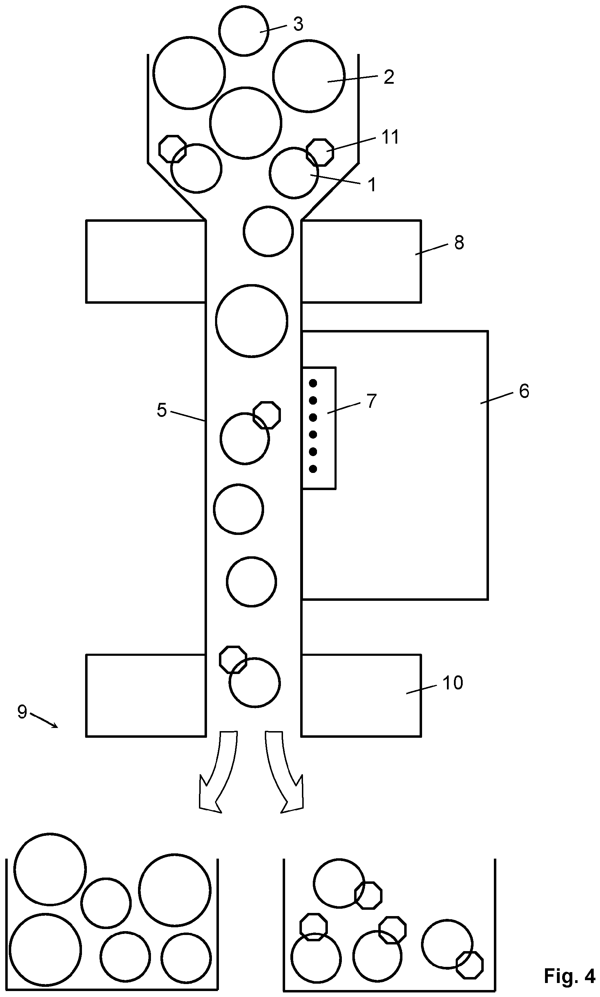

Diese Mischung aus Zellen verschiedener Zellarten wird anschließend einer Vorrichtung zur Durchführung einer Magnetfeldmessung an mit magnetischen Nanopartikeln markierten Zellen einer Biopsieprobe zugeführt. Diese Vorrichtung ist in einer schematischen Schnittansicht in Fig. 4 dargestellt.

Fig. 4 zeigt Zellen einer ersten Zellart 1, einer zweiten Zellart 2 und einer dritten Zellart 3, wobei in der Figur lediglich jeweils eine der Zellen mit einem Bezugszeichen versehen ist. Die Zellen der ersten Zellart 1 sind mit ersten Nanopartikeln 11 markiert. Diese ersten Nanopartikel 11 sind aus zeichnerischen Gründen als Achtecke dargestellt. In Realität weisen die ersten Nanopartikel 11 jedoch eine andere Gestalt auf. Auch ist in der Figur jeweils nur ein erstes Nanopartikel 11 mit einer Zelle der ersten Zellart 1 verbunden. Es ist jedoch auch möglich, dass mehrere erste Nanopartikel 11 an einer ersten Zelle der ersten Zellart 1 angelagert sind.

Die Vorrichtung zur Durchführung einer Magnetfeldmessung weist einen Mikrokanal 5 auf. Der Mikrokanal 5 besitzt einen Querschnitt aufweist, der den gleichzeitigen Durchgang lediglich einer der markierten Zellen der ersten Zellart 1 erlaubt. Typischerweise liegt der Durchmesser des Mikrokanals 5 im Bereich 10 Mikrometer bis 100 Mikrometer, ist jedoch abhängig von den zu detektieren Zellarten sowie von den restlichen Zellarten der Biopsieprobe.

Ferner weist die Vorrichtung zur Durchführung einer Magnetfeldmessung einen auf dem Farbzentrum-Prinzip basierenden Quantenmagnetfeldsensor 6 auf. Der Quantenmagnetfeldsensor 6 wiederum besitzt eine Sensorschicht 7, in der Farbzentren ausgebildet sind. In der beschriebenen Ausführungsform handelt es sich hierbei um NV(Stickstoff-Fehlstellen)-Zentren im Diamant, bevorzugt mit einer NV-Zentren-Dichte von 0,1 ppm bis 100 ppm. Es sind jedoch auch andere Farbzentren möglich, z.B.: Silizium-Fehlstellen-Zentren (SiV), Germanium-Fehlstellen-Zentren (GeV), Nickel-Fehlstellen-Zentren (NiV), Chrom-Fehlstellen-Zentren (CrV), Silber-Fehlstellen-Zentren (AgV), Zinn-Fehlstellen-Zentren oder C60-Farbzentren (Fullerene).

Die Sensorschicht 7 bildet einen Teilbereich einer inneren Oberfläche des Mikrokanals 5. Am Eingang des Mikrokanals 5 ist ein Durchflussregler 8 angeordnet und am Ende des Mikrokanals 5 ist als Bestandteil einer Aussortiervorrichtung 9 ein Mikrofluidschalter 10 positioniert.

An den Zellen, die den Durchflussregler 8 passiert haben, wird im Mikrokanal 5 einer Magnetfeldmessung mittels des Quantenmagnetfeldsensors 6 durchgeführt. Bei dem hierbei durchgeführten Messverfahren kann es sich um eine Continous Wave-Messung, insbesondere um eine ODMR(Optically Detected Magnetic Resonance)-Messung, oder um eine gepulste Messung handeln. Beispiele für Continous Wave-Messverfahren wären, neben ODMR, beispielsweise auch: Continuous Wave Electron Paramagnetic Resonance (CW-EPR), Continuous Wave Nuclear Magnetic Resonance (CW-NMR), Continuous Wave Electron Spin Resonance (CW-ESR), Continuous Wave Photoacoustic Spectroscopy (CW-PAS). Beispiele für gepulste Messverfahren wären beispielsweise: Pulsed Electron Paramagnetic Resonance (Pulsed EPR), Pulsed Nuclear Magnetic Resonance (Pulsed NMR), Dynamical Decoupling, Ramsey Interferometry, Spin Echo, Carr-Purcell-Meiboom-Gill (CPMG) Sequenz, Rabi Oscillations.

Durch die Magnetfeldmessung wird bestimmt, ob die den Quantenmagnetfeldsensor 6 passierende Zelle eine mit einem ersten magnetischen Nanopartikel 11 markierte Zelle der ersten Zellart 1 oder eine nichtmarkierte Zelle (d.h. eine Zelle der zweiten Zellart 2 oder der dritten Zellart 3). In Abhängigkeit vom Ergebnis des Quantenmagnetfeldsensors 6 sortiert die Aussortiervorrichtung 9 die Zelle entsprechend. Mit anderen Worten trennt die Aussortiervorrichtung 9 die markierten Zellen der ersten Zellart 1 von den restlichen Zellarten 2, 3 der Biopsieprobe und sortiert auf diese Weise die Zellen der ersten Zellart 1 aus. Diese Aussortierung ist in Figur 4 durch das Sammeln der Zellen der ersten Zellart 1 in dem rechten Behälter und das Sammeln der nichtmarkierten Zellen 2, 3 im linken Behälter schematisch dargestellt.

In Figur 1 nicht dargestellt ist eine anschließende lichtmikroskopische Untersuchung der aussortierten Zellen der ersten Zellart 1, um diese Zellen näher zu bestimmen.

Die oben beschriebene Vorrichtung zur Durchführung einer Magnetfeldmessung kann Bestandteil eines (in den Figuren nicht dargestellten) Systems zur Detektion von Zellen der ersten Zellart 1 in der Biopsieprobe sein. Dieses System weist des Weiteren eine Gewebedissoziationseinrichtung zum Durchführen der Gewebedissoziation der Biopsieprobe, eine Markiereinrichtung zum Zuführen der magnetischen Nanopartikeln 11 zu den vereinzelten Zellen und zum selektiven Markieren der Zellen der ersten Zellart 1, eine Entfernungseinrichtung zum Entfernen der überflüssigen magnetischen Nanopartikel 11 sowie die Aussortiervorrichtung 9 zum Aussortieren der markierten Zellen der ersten Zellart 1 nach dem Durchlaufen des Mikrokanals 5 auf.

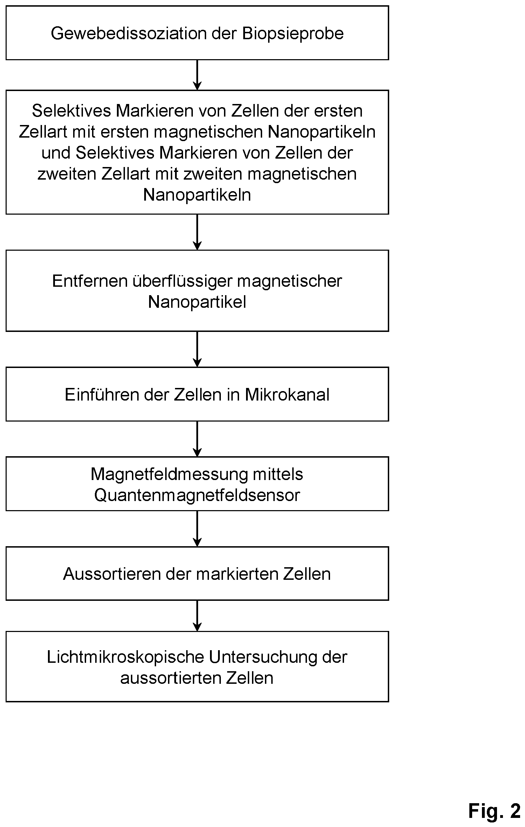

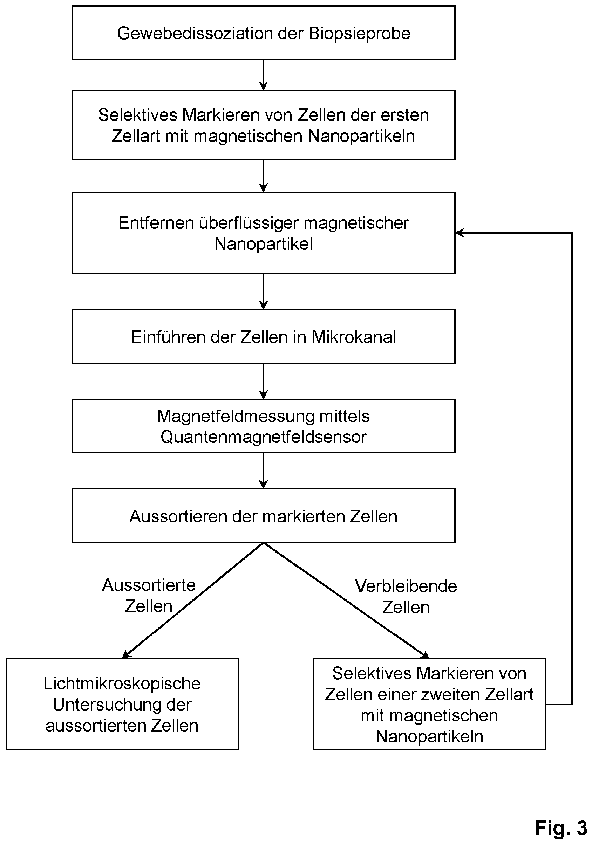

Figur 2 und Figur 3 zeigen Verfahrensvarianten, die die Aufteilung der Gewebeprobe in mehr als zwei Fraktionen erlauben. Mit anderen Worten können mit diesen Verfahren nicht nur Zellen einer ersten Zellart 1, sondern beispielsweise auch Zellen einer zweiten Zellart 2 aussortiert werden.

Das in Figur 2 dargestellte Verfahren unterscheidet sich von dem in Figur 1 dargestellten Verfahren dadurch, dass im Verfahrensschritt "Zuführen von magnetischen Nanopartikeln" erste magnetische Nanopartikel 11 zum selektiven Markieren der Zellen der ersten Zellart 1 und zweite magnetische Nanopartikel 12 zum selektiven Markieren von Zellen einer zweiten Zellart 2 zugeführt werden. Die ersten magnetischen Nanopartikel 11 unterscheiden sich von den zweiten Nanopartikeln 12 hierbei sowohl in ihrer Oberflächenbeschaffenheit als auch in ihren magnetischen Eigenschaften. Während der Magnetfeldmessung im Mikrokanal 5 kann dann zwischen den markierten Zellen der ersten Zellart 1 und den markierten Zellen der zweiten Zellart 2 unterschieden werden. Im Ergebnis kann ein gleichzeitiges, selektives Aussortieren der Zellen der ersten Zellart 1 und den Zellen der zweiten Zellart 2 erfolgen.

An den aussortierten Zellen der ersten Zellart 1 und der zweiten Zellart 2 werden anschließend lichtmikroskopische Untersuchungen durchgeführt, um diese Zellen näher zu bestimmen.

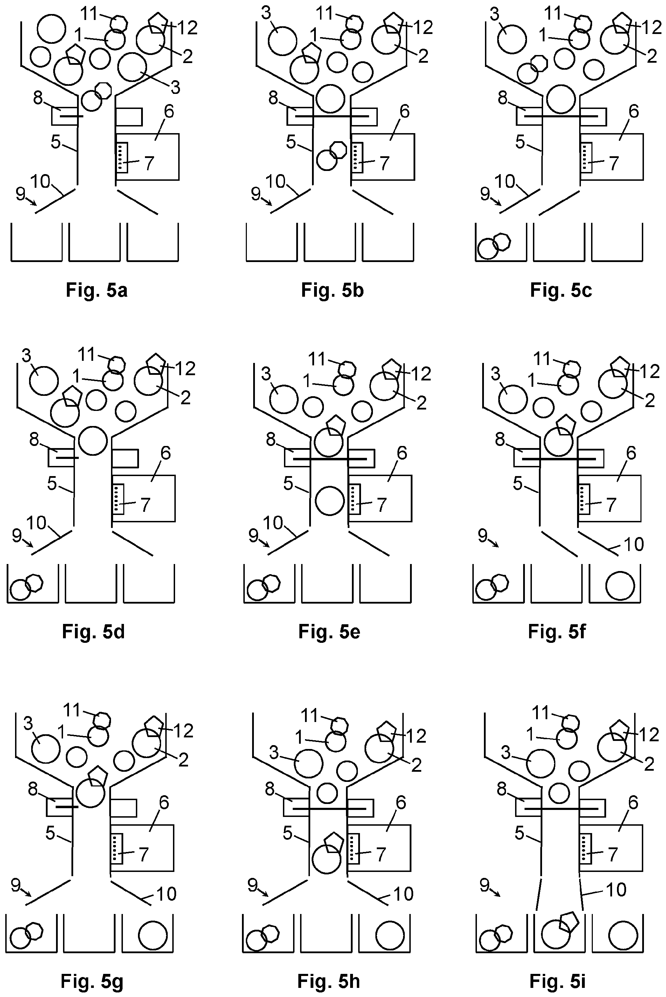

Figur 5a bis Figur 5i zeigen schematisch den Ablauf der Sortierung von Zellen einer Biopsieprobe nach dem in Figur 2 dargestellten Verfahren. Der Vorrichtung zur Durchführung einer Magnetfeldmessung wird eine dissoziierte Biopsieprobe mit Zellen der ersten Zellart 1, die mit ersten magnetischen Nanopartikeln 11 markiert sind, Zellen der zweiten Zellart 2, die mit zweiten magnetischen Nanopartikeln 12 markiert sind, und unmarkierte Zellen einer dritten Zellart 3 zugeführt. Die zweiten Nanopartikel 12 sind aus zeichnerischen Gründen als Fünfecke dargestellt.

In Realität weisen die zweiten Nanopartikel 12 jedoch eine andere Gestalt auf. Auch ist in der Figur jeweils nur ein zweites Nanopartikel 12 mit einer Zelle der zweiten Zellart 2 verbunden. Es ist jedoch auch möglich, dass mehrere zweite Nanopartikel 12 an einer Zelle der zweiten Zellart 2 angelagert sind.

Figur 5a zeigt einen Zustand, in dem der Durchflussregler 8 geöffnet wird und eine markierte Zelle der ersten Zellart 1 in den Mikrokanal 5 eintritt. Die mit dem ersten Nanopartikel 11 markierte Zelle der ersten Zellart 1 bewegt sich entlang des Mikrokanals 5 und passiert hierbei den Quantenmagnetfeldsensor 6 mit dessen Sensorschicht 7 (vgl. Figur 5b). Der Quantenmagnetfeldsensor 6 detektiert das durch das erste magnetische Nanopartikel 11 erzeugte Magnetfeld. Basierend auf diesem Messergebnis ordnet die Aussortiervorrichtung 9 die markierte Zelle der ersten Zellart 1 einer ersten Fraktion zu und steuert den Mikrofluidschalter 10 derart, dass die markierte Zelle der ersten Zellart 1 in einem ersten Behälter gesammelt wird (vgl. Figur 5c).

Figur 5b zeigt einen Zustand, in dem der Durchflussregler 8 geöffnet wird und eine nichtmarkierte Zelle der dritten Zellart 3 in den Mikrokanal 5 eintritt. Diese nichtmarkierte Zelle bewegt sich entlang des Mikrokanals 5 und passiert hierbei ebenfalls den Quantenmagnetfeldsensor 6 mit dessen Sensorschicht 7 (vgl. Figur 5e). Der Quantenmagnetfeldsensor 6 detektiert keine bzw. keine signifikante Magnetfeldänderung. Basierend auf diesem Messergebnis ordnet die Aussortiervorrichtung 9 die Zelle der dritten Zellart 3 einer anderen Fraktion (z.B. einer dritten Fraktion) zu und steuert den Mikrofluidschalter 10 derart, dass die Zelle der dritten Zellart 3 in einem dritten Behälter gesammelt wird (vgl. Figur 5f).

Figur 5g zeigt einen Zustand, in dem der Durchflussregler 8 geöffnet wird und eine markierte Zelle der zweiten Zellart 2 in den Mikrokanal 5 eintritt. Die mit dem zweiten Nanopartikel 12 markierte Zelle der zweiten Zellart 2 bewegt sich entlang des Mikrokanals 5 und passiert hierbei den Quantenmagnetfeldsensor 6 mit dessen Sensorschicht 7 (vgl. Figur 5h). Der Quantenmagnetfeldsensor 6 detektiert das durch das zweite magnetische Nanopartikel 12 erzeugte Magnetfeld. Basierend auf diesem Messergebnis ordnet die Aussortiervorrichtung 9 die markierte Zelle der zweiten Zellart 2 einer zweiten Fraktion zu und steuert den Mikrofluidschalter 10 derart, dass die markierte Zelle der zweiten Zellart 2 in einem zweiten Behälter gesammelt wird (vgl. Figur 5i).

Figur 3 zeigt eine weitere Verfahrensvariante, die die Aufteilung der Gewebeprobe in mehr als zwei Fraktionen erlaubt. Das in Figur 3 dargestellte Verfahren unterscheidet sich von dem in Figur 1 dargestellten Verfahren dadurch, dass die nach dem Verfahrensschritt "Aussortieren der markierten Zellen" verbleibenden Zellen wieder die Verfahrensschritte "Zuführen von magnetischen Nanopartikeln" bis "Aussortieren der markierten Zellen" durchgeführt werden, wobei im Verfahrensschritt "Zuführen von magnetischen Nanopartikeln" dann weitere magnetische Nanopartikel (insbesondere zweite magnetische Nanopartikel 12) zum selektiven Markieren von Zellen einer weiteren Zellart (nämlich zum Markieren der Zellen der zweiten Zellart 2) zugeführt werden. Im Ergebnis kann ein serielles, selektives Aussortieren der Zellen der ersten Zellart 1 und der Zellen der zweiten Zellart 2 erfolgen. Die jeweils aussortierten Zellen werden anschließend lichtmikroskopische Untersuchungen durchgeführt, um diese Zellen näher zu bestimmen.

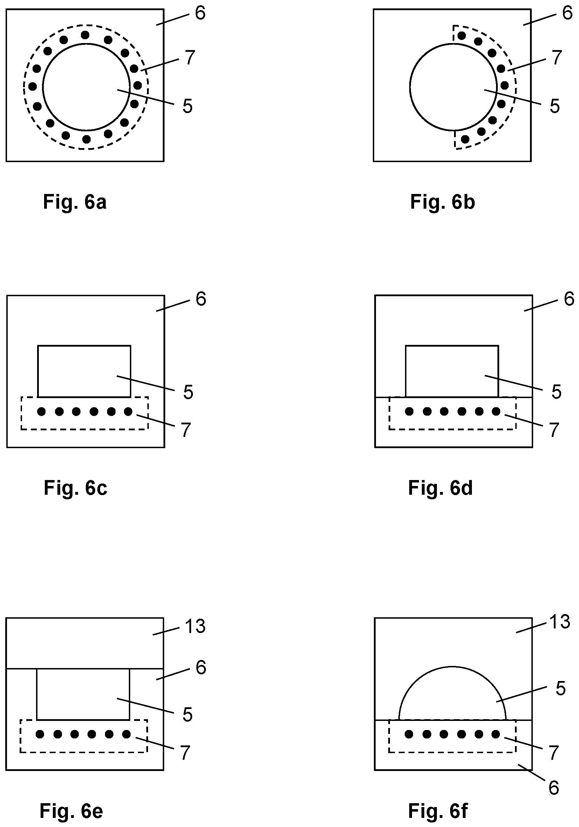

Der Querschnitt des Mikrokanals 5 ist derart ausgebildet, dass dieser den gleichzeitigen Durchgang lediglich einer markierten Zelle der ersten Zellart 1 erlaubt. Die Figuren 6a bis 6f zeigen schematische Querschnittsdarstellungen des Mikrokanals 5 mit Quantenmagnetfeldsensor 6. Der Quantenmagnetfeldsensor 6 weist jeweils ein Sensorschicht 7 auf, in der die Farbzentren (in den Figuren schematisch als Punkte visualisiert) ausgebildet sind.

Figur 6a zeigt einen zylinderförmigen Mikrokanal 5, dessen Oberfläche durch die Sensorschicht 7 des Quantenmagnetfeldsensors 6 gebildet wird. Ein derartiger Mikrokanal 5 kann etwa durch Bohren oder Ätzen in das Grundmaterial (z.B. Diamant) des Quantenmagnetfeldsensors 6 hergestellt werden.

Figur 6b zeigt eine weitere Ausführungsform eines zylindrischen Mikrokanals 5, wobei hier lediglich ein Teilbereich (die Hälfte der Mantelfläche) der Oberfläche des Mikrokanals 5 durch die Sensorschicht 7 des Quantenmagnetfeldsensors 6 gebildet wird.

Figur 6c zeigt eine Ausführungsform eines Mikrokanals 5 mit rechteckigem Querschnitt. Eine Seite des Rechtecks bildet die Sensorschicht 7 des Quantenmagnetfeldsensors 6. Die restlichen Seiten des Rechtecks bildet das Grundmaterial des Quantenmagnetfeldsensors 6. Die Herstellung eines derartigen Mikrokanals 5 kann beispielsweise durch Ätzen des Grundmaterials erfolgen.

Figur 6d zeigt eine Ausführungsform eines Mikrokanals 5 mit rechteckigem Querschnitt, wobei der Mikrokanal 5 zunächst auf der Oberfläche des Grundmaterials des Quantenmagnetfeldsensors 6 ausgebildet wurde (insbesondere durch mechanische Bearbeitung wie Fräsen oder durch Ätzen). Der auf diese Weise entstandene offene Kanal wird anschließen durch eine separat hergestellte Sensorschicht 7 verschlossen.

Figur 6e zeigt eine weitere Ausführungsform eines Mikrokanals 5 mit rechteckigem Querschnitt, wobei der Mikrokanal 5 zunächst auf der Oberfläche des Grundmaterials des Quantenmagnetfeldsensors 6 ausgebildet wurde. Der auf diese Weise entstandene offene Kanal wird anschließen durch einen separat hergestellten Deckel 13 verschlossen. Die Sensorschicht 7 ist im Grundmaterials des Quantenmagnetfeldsensors 6 ausgebildet und bildet den Nutgrund des Mikrokanals 5.

Figur 6f zeigt eine Ausführungsform eines Mikrokanals 5 mit halbrundem Querschnitt, wobei ein Deckel 13 mit halbrunder Innenform die Sensorschicht 7 des Quantenmagnetfeldsensors 6 abdeckt.

### BEZUGSZEICHENLISTE

- 1: Zelle einer ersten Zellart
- 2: Zelle einer zweiten Zellart
- 3: Zelle einer dritten Zellart
- 5: Mikrokanal
- 6: Quantenmagnetfeldsensor
- 7: Sensorschicht mit Farbzentren
- 8: Durchflussregler
- 9: Aussortiervorrichtung
- 10: Mikrofluidschalter
- 11: Magnetische Nanopartikel erster Art
- 12: Magnetische Nanopartikel zweiter Art
- 13: Deckel

## Patentansprüche

1. Verfahren zur Detektion von Zellen einer ersten Zellart (1) in einer Biopsieprobe, in der eine Mehrzahl von Zellen unterschiedlicher Zellarten (2, 3) vorhanden sind, mit folgenden Verfahrensschritten in der angegebenen Reihenfolge:
- Durchführen einer Gewebedissoziation der Biopsieprobe zum Vereinzeln der in der Biopsieprobe enthaltenen Zellen;
- Zuführen von magnetischen Nanopartikeln (11) zu den vereinzelten Zellen zum selektiven Markieren der Zellen der ersten Zellart (1);
- Entfernen der überflüssigen magnetischen Nanopartikel (11);
- Einführen der Zellen in einen Mikrokanal (5), wobei der Mikrokanal (5) einen Querschnitt aufweist, der den gleichzeitigen Durchgang lediglich einer markierten Zelle (1) erlaubt;
- Durchführen einer Magnetfeldmessung im Mikrokanal (5) mittels eines auf dem Farbzentrum-Prinzip basierenden Quantenmagnetfeldsensors (6); und
- Aussortieren der markierten Zellen (1) nach dem Durchlaufen des Mikrokanals (5).

2. Verfahren nach Anspruch 1, wobei der Quantenmagnetfeldsensor (6) eine Sensorschicht (7) aufweist, in der Farbzentren ausgebildet sind und die zumindest einen Teilbereich einer inneren Oberfläche des Mikrokanals (5) bildet.

3. Verfahren nach einem der Ansprüche 1 oder 2, wobei die magnetischen Nanopartikel (11) als superparamagnetische Eisenoxid-Nanopartikel ausgebildet sind.

4. Verfahren nach einem der Ansprüche 1 bis 3 ferner aufweisend den folgenden Verfahrensschritt: Verändern des Querschnitts des Mikrokanals (5) in Abhängigkeit von der Größe der Zellen.

5. Verfahren nach einem der Ansprüche 1 bis 4, wobei die Biopsieprobe eine Feinnadelbiopsieprobe ist.

6. Verfahren nach einem der Ansprüche 1 bis 5, wobei die Magnetfeldmessung eine Continous Wave-Messung ist.

7. Verfahren nach einem der Ansprüche 1 bis 5, wobei die Magnetfeldmessung als gepulste Messung durchgeführt wird.

8. Verfahren nach einem der Ansprüche 1 bis 7, wobei nach dem Verfahrensschritt "Aussortieren der markierten Zellen" als weiterer Verfahrensschritt eine lichtmikroskopische Untersuchung der aussortierten, markierten Zellen (1) durchgeführt wird.

9. Verfahren nach einem der Ansprüche 1 bis 8, wobei im Verfahrensschritt "Zuführen von magnetischen Nanopartikeln" erste magnetische Nanopartikel (11) zum selektiven Markieren der Zellen der ersten Zellart (1) und zweite magnetische Nanopartikel (12) zum selektiven Markieren von Zellen einer zweiten Zellart (2) zugeführt werden, und
wobei im Verfahrensschritt "Aussortieren der markierten Zellen" ein Aussortieren der mit den ersten magnetischen Nanopartikeln (11) und der mit den zweiten magnetischen Nanopartikeln (12) markierten Zellen (1, 2) erfolgt.

10. Verfahren nach einem der Ansprüche 1 bis 9, wobei an den nach dem Verfahrensschritt "Aussortieren der markierten Zellen" verbleibenden Zellen wieder die Verfahrensschritte "Zuführen von magnetischen Nanopartikeln" bis "Aussortieren der markierten Zellen" durchgeführt werden, wobei im Verfahrensschritt "Zuführen von magnetischen Nanopartikeln" dann weitere magnetische Nanopartikel zum selektiven Markieren von Zellen einer weiteren Zellart zugeführt werden.

11. Vorrichtung zur Durchführung einer Magnetfeldmessung an mit magnetischen Nanopartikeln (11) markierten Zellen (1) einer Biopsieprobe, aufweisend:
- einen Mikrokanal (5), wobei der Mikrokanal (5) einen Querschnitt aufweist, der den gleichzeitigen Durchgang lediglich einer markierten Zelle (1) erlaubt;
- einen auf dem Farbzentrum-Prinzip basierenden Quantenmagnetfeldsensor (6) zur Durchführung einer Magnetfeldmessung im Mikrokanal (5).

12. Vorrichtung nach Anspruch 11, wobei der
Quantenmagnetfeldsensor (6) eine Sensorschicht (7) aufweist, in der Farbzentren ausgebildet sind und die zumindest einen Teilbereich einer inneren Oberfläche des Mikrokanals (5) bildet.

13. Vorrichtung nach einem der Ansprüche 11 oder 12, wobei der Mikrokanal (5) einen veränderbaren Querschnitt aufweist.

14. Vorrichtung nach einem der Ansprüche 11 bis 13 mit einem Durchflussregler (8) am Eingang des Mikrokanals (5).

15. System zur Detektion von Zellen einer ersten Zellart (1) in einer Biopsieprobe, in der eine Mehrzahl von Zellen unterschiedlicher Zellarten vorhanden sind, aufweisend:
- eine Gewebedissoziationseinrichtung zum Durchführen einer Gewebedissoziation der Biopsieprobe zum Vereinzeln der in der Biopsieprobe enthaltenen Zellen;
- eine Markiereinrichtung zum Zuführen von magnetischen Nanopartikeln (11) zu den vereinzelten Zellen und zum selektiven Markieren der Zellen der ersten Zellart (1);
- eine Entfernungseinrichtung zum Entfernen der überflüssigen magnetischen Nanopartikel (11);
- eine Vorrichtung nach einem der Ansprüche 11 bis 14; und
- eine Aussortiervorrichtung (9) zum Aussortieren der markierten Zellen (1) nach dem Durchlaufen des Mikrokanals (5).

16. System nach Anspruch 15, wobei die Aussortiervorrichtung (9) einen am Ausgang des Mikrokanals (5) angeordneten Mikrofluidschalter (10) aufweist.
